# EUROPÄISCHE PATENTANMELDUNG

(11) **EP 3 964 506 A1**
(43) Veröffentlichungstag der Anmeldung: **09.03.2022**
(21) Anmeldenummer: 20194636.5
(22) Anmeldetag: 04.09.2020
(51) Int. Cl.: C07D 401/14

(54) **VERFAHREN ZUR HERSTELLUNG EINES TETRAZOL-SUBSTITUIERTEN ANTHRANILSÄUREDIAMID-DERIVATES**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein Verfahren zur Herstellung eines tetrazol-substituierten Anthranilsäurediamid-Derivates gemäß der Formel (I) 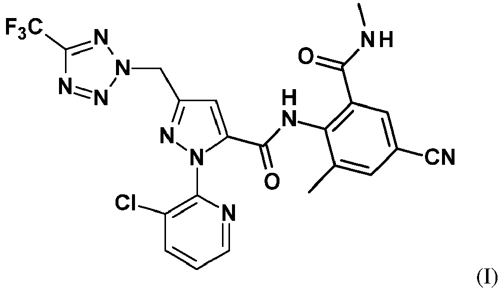 in kristalliner Form, über Solvat Kristalle, in hoher Reinheit und Ausbeute. Die vorliegende Erfindung betrifft weiterhin auch die Bereitstellung neuer Solvat Kristalle, die sich im Vergleich zu einem tetrazol-substituierten Anthranilsäurediamid-Derivat gemäß oben genannter Formel (I) in kristalliner Form durch verbesserte Filtrationseigenschaften auszeichnen.

## Beschreibung

Die vorliegende Erfindung betrifft ein Verfahren zur Herstellung eines tetrazol-substituierten Anthranilsäurediamid-Derivates gemäß der Formel (I) 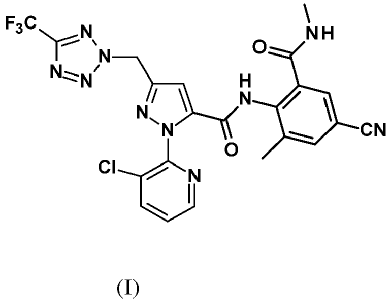 in kristalliner Form, über Solvat-Kristalle, in hoher Reinheit und Ausbeute. Die vorliegende Erfindung betrifft weiterhin auch die Bereitstellung neuer Solvat-Kristalle, die sich im Vergleich zu einem tetrazol-substituierten Anthranilsäurediamid-Derivat gemäß oben genannter Formel (I) in kristalliner Form durch verbesserte Filtrationseigenschaften auszeichnen.

In der WO2011/157664 A1 ist ein Verfahren zur Herstellung von tetrazol-substituierten Anthranilsäurediamid-Derivaten u.a. auch gemäß oben genannter Formel (I) beschrieben. Weiterhin ist in WO2011/157664 A1 ein Verfahren zur Herstellung einer kristallinen Form eines tetrazol-substituierten Anthranilsäurediamid-Derivates gemäß oben genannter Formel (I) beschrieben, welches aufgrund seiner physikochemischen Eigenschaften gut zu handhaben ist und die Produktion einer stabilen Formulierung ermöglicht.

Nachteilig am in WO2011/157664 A1 beschriebenen Verfahren sind die physikalischen Eigenschaften der dort beschriebenen kristallinen Form eines tetrazol-substituierten Anthranilsäurediamid-Derivates gemäß oben genannter Formel (I) hinsichtlich seiner Filtrationseigenschaften. Nach dem in WO2011/157664 A1 beschriebenen Verfahren fällt die Verbindung gemäß oben genannter Formel (I) in Form von feinen Nadeln an, die bei der Isolierung durch Filtration lange Filtrationszeiten und hohe Restfeuchten im Filterkuchen verursachen. Die hohen Restfeuchten im Filterkuchen führen darüber hinaus zu langen Trocknungszeiten des Feuchtguts. Aufgrund der beschriebenen Probleme ist das in WO2011/157664 A1 beschriebene Verfahren zur Herstellung eines tetrazol-substituierten Anthranilsäurediamid-Derivates gemäß oben genannter Formel (I) im großtechnischen Produktionsmaßstab nicht zweckmäßig.

Aufgabe der vorliegenden Erfindung ist daher die Bereitstellung eines neuen Verfahrens zur Herstellung eines tetrazol-substituierten Anthranilsäurediamid-Derivates gemäß oben genannter Formel (I) in kristalliner Form mit hoher Reinheit und Ausbeute, welches auch im großtechnischen Produktionsmaßstab unter Einbeziehung von wirtschaftlichen Gesichtspunkten vorteilhaft ist.

Die Aufgabe wurde gemäß der vorliegenden Erfindung gelöst durch ein Verfahren zur Herstellung einer Verbindung der Formel (I) 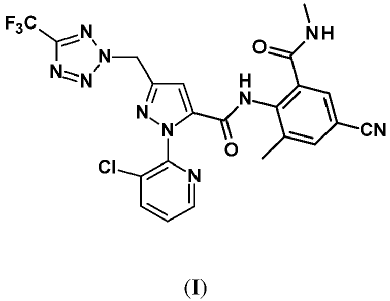 in kristalliner Form, dadurch gekennzeichnet, dass eine Verbindung der Formel (I) in mindestens einem amidischen Lösungsmittel gelöst wird und durch das Vorhandensein von mindestens einem Antilösungsmittel und/oder durch Temperaturerniedrigung zu einem Solvat kristallisiert und anschließend filtriert und getrocknet wird. Überraschend ist dabei insbesondere auch, dass durch Trocknung der Solvat-Kristalle die Verbindung der Formel (I) in kristalliner Form erhalten werden kann.

Bevorzugt handelt es sich bei der gemäß dem erfindungsgemäßen Verfahren erhaltenen kristallinen Form der Verbindung der Formel (I) um die thermodynamisch stabile Kristallform.

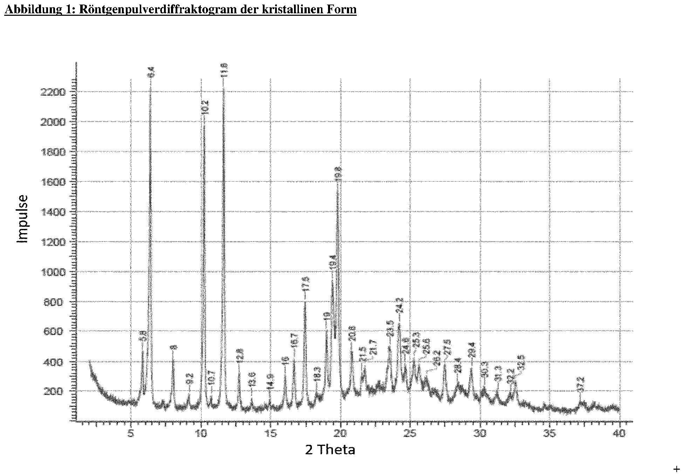

Ebenfalls bevorzugt wird nach dem erfindungsgemäßen Verfahren eine Verbindung der Formel (I) in einer kristallinen Form erhalten, die ein charakteristisches Röntgenpulverdiffraktogramm, Raman-Spektrum und IR-Spektrum (Tabelle 1 und 2, Abb. 1, 2, 3) aufweist. Eine Verbindung der oben genannten Formel (I) in dieser kristallinen Form ist dadurch gekennzeichnet, dass ihr Röntgenpulverdiffraktogramm bei einer Temperatur von 25°C und bei Verwendung von Cu Kα Strahlung mindestens folgende Reflexlagen (2 Theta) aufweist: 5.8°, 6.4°, 11.6°, 17.5°, 19.8°, 20.8°, 23.5° und 24.2° (jeweils ± 0,2°). Vorzugsweise weist das Röntgenpulverdiffraktogramm der kristallinen Form bei einer Temperatur von 25°C und bei Verwendung von Cu Kα-Strahlung mindestens folgende weitere Reflexlagen (2 Theta) auf: 10.2°, 12.8°, 16.7°, 19.0°, 25.3°, 27.5°, 29.4° (jeweils ± 0,2°). In einer weiteren bevorzugten Variante der vorliegenden Erfindung entspricht das Röntgenpulverdiffraktogramm der kristallinen Form bei einer Temperatur von 25°C und Verwendung von Cu Kα-Strahlung im Wesentlichen dem in Abbildung 1 wiedergegebenen Diffraktogramm.

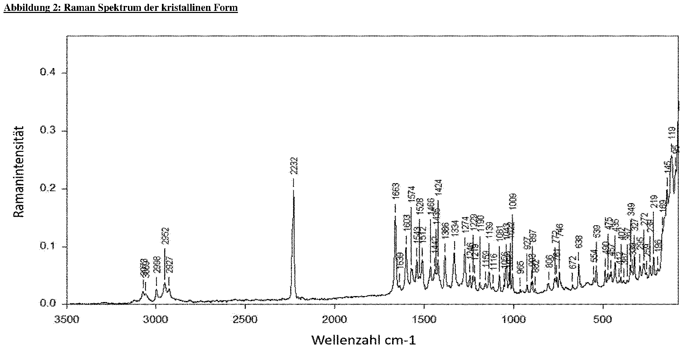

Besonders bevorzugt weist das Raman-Spektrum der kristallinen Form mindestens folgende Banden [cm⁻¹] auf: 2927, 1663, 1386, 1334, 1022, 638 (jeweils± 2° cm⁻¹). In einer weiteren besonders bevorzugten Ausführungsform der Erfindung entspricht das Raman-Spektrum der kristallinen Form im Wesentlichen dem in Abbildung 2 wiedergegebenen Spektrum.

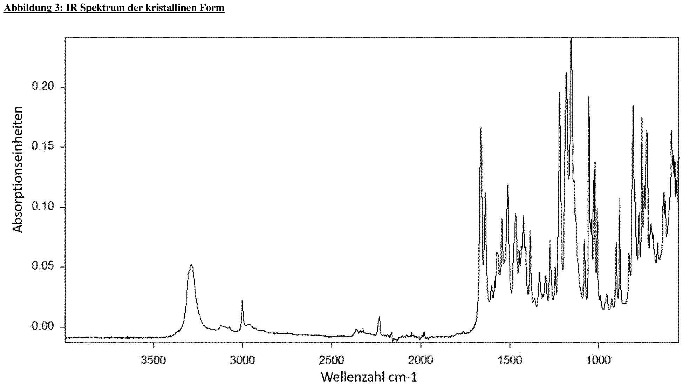

Besonders bevorzugt weist das IR-Spektrum der kristallinen Form mindestens folgende Banden [cm⁻¹] auf: 3286, 1662, 1219, 1181, 1154, 1055 (jeweils± 2° cm⁻¹). In einer weiteren besonders bevorzugten Ausführungsform der Erfindung entspricht das IR-Spektrum der kristallinen Form im Wesentlichen dem in Abbildung 3 wiedergegebenen Spektrum.

Alle Röntgenpulverdiffraktometrie-Daten der kristallinen Form wurden mit folgenden Akquisitionsparametern bei 25°C erhalten:

| | |
|---|---|
| Diffraktometer Typ: | PANalytic X'Pert PRO |
| Anodenmaterial: | Cu |
| Strahlung: | Cu Kα 1 |
| Wellenlänge : | 1.54060 Å |
| Scan Modus: | Transmission |
| Scan Typ: | 2Theta: Omega |
| Angabe: | 2 Theta (Peakmaximum) ± 0,2° |

Die Raman-Spektren der kristallinen Form wurden mit FT-Ramanspektrometern der Firma Bruker (z.B. mit den Modellen RFS100 oder MultiRam) bei 25°C und mit einer Laserwellenlänge von 1064 nm und einer Auflösung von 2 cm⁻¹ aufgenommen.

Die IR-Spektren der kristallinen Form wurden mit IR-Spektrometern der Firma Bruker (z.B. mit dem Modell Tensor 37) mit einer universellen Diamant ATR-Einheit bei 25°C und mit einer Auflösung von 4 cm⁻¹ aufgenommen.
Abbildung 1 zeigt das Röntgenpulverdiffraktogram der kristallinen Form
Abbildung 2 zeigt das Raman Spektrum der kristallinen Form
Abbildung 3 zeigt das IR Spektrum der kristallinen Form
Abbildung 4 zeigt das Röntgenpulverdiffraktogram des DMAc Solvats
Abbildung 5 zeigt das Röntgenpulverdiffraktogram des NMP Solvats
Abbildung 6 zeigt das Raman Spektren des DMAc und NMP Solvats
Abbildung 7 zeigt das IR Spektrum des DMAc Solvats
Abbildung 8 zeigt das IR Spektrum des NMP Solvats

Ausgangspunkt für die Herstellung einer Verbindung der oben genannten Formel (I) in einer kristallinen Form gemäß dem vorliegenden Verfahren ist das Vorhandensein einer Eduktmischung bei der eine Verbindung der oben genannten Formel (I) in mindestens einem amidischen Lösungsmittel ausgewählt aus der Gruppe von N,N-Dimethylformamid, N,N-Dimethylacetamid, N-Methylformanilid, N-Methyl-2-pyrrolidon, N-Methylcaprolactam und Hexamethylphosphorsäuretriamid und ganz besonders bevorzugt ausgewählt aus der Gruppe von N-N-Dimethylacetamid und N-Methyl-2-pyrrolidon gelöst ist. In einer weiteren bevorzugten Ausführungsform der Erfindung liegt ein amidisches Lösungsmittel vor und kein Gemisch der oben genannten amidischen Lösungsmittel.

Bei der Verwendung von N-N-Dimethylacetamid wird das weiter unten beschriebene kristalline N-N-Dimethylacetamid Solvat der Verbindung der Formel (I) (DMAc Solvat) erhalten. Bei der Verwendung von N-Methyl-2-pyrrolidon wird das weiter unten beschriebene kristalline N-Methyl-2-pyrrolidon Solvat der Verbindung der Formel (I) (NMP Solvat) erhalten.

Im Vergleich zu der Verbindung der Formel (I) in kristalliner Form weist das DMAc Solvat und das NMP Solvat deutlich verbesserte Filtrationseigenschaften auf (siehe Tabelle 5). Der in der Tabelle 5 gemessene Filtrationswiderstand α wurde gemessen durch die Aufnahme einer Druckfiltrationskurve. Der Filtrationsversuch wurde in einer Apparatur zur Bestimmung des spezifischen Filtrationswiderstands (alpha-Wert) durchgeführt, entsprechend der VDI Richtlinie 2762 (Hersteller: BHS Sonthofen, Modell: Taschenmessgerät KPL TMG 400). Dafür wurde die zu bestimmende Suspension in die Apparatur eingefüllt und durch Anlegen einer Druckdifferenz die anfallende Filtratmenge (Mutterlauge) kontinuierlich gemessen. Die Filtratmenge V wird als Funktion der Zeit t aufgezeichnet (Filtratkurve). Durch Auftragen von t/V gegen V kann der spezifische Filtrationswiderstand (alpha-Wert in m⁻²) ermittelt werden (siehe auch W. Beckmann: Crystallization - Basic Concepts and Industrial Applications, Wiley-VCH, 2013, Kap. 14.2.3).

Das mindestens eine amidische Lösungsmittel wird vorzugsweise in einem 2- bis 20-fachen Gewichtsüberschuß, besonders bevorzugt in einem 3- bis 10-fachen Gewichtsüberschuß bezogen auf die Verbindung der Formel (I) in der Eduktmischung eingesetzt.

Eine Verbindung der Formel (I) in mindestens einem amidischen Lösungsmittel kann durch Temperaturerniedrigung zum Solvat, ohne Zugabe von mindestens einem Antilösungsmittel, kristallisiert werden. Die Temperaturerniedrigung erfolgt dabei vorzugsweise langsam, besonders bevorzugt in einem Bereich von 1 bis 10 °C / Stunde und noch bevorzugter in einem Bereich von 1 bis 5°C / Stunde in dem weiter unten angegebenen Temperaturbereich. Die Verbindung der Formel (I) in mindestens einem amidischen Lösungsmittel liegt dabei vorzugsweise in einer homogenen Mischung vor, die beispielsweise durch Rühren erreicht werden kann.

Für das erfindungsgemäße beschriebene Verfahren wird in einer bevorzugten Ausführungsform der Erfindung mindestens ein Antilösungsmittel eingesetzt, im Wesentlichen um die Löslichkeit herab zu setzten und eine höhere Ausbeute des Solvats zu erzielen. Die Kristallisation zum Solvat kann dann auch ohne Temperaturerniedrigung bzw. insgesamt auch bei höherer Verfahrenstemperatur erfolgen. In einer besonders bevorzugten Ausführungsform der Erfindung wird für die Kristallisation zum Solvat das Vorhandensein bzw. die Zugabe von mindestens einem Antilösungsmittel und die (vorzugsweise anschließende) Temperaturerniedrigung miteinander kombiniert. Als mindestens ein Antilösungsmittel wird vorzugsweise ein Antilösungsmittel ausgewählt aus der Gruppe von Wasser, Acetonitril, C₁-C₆-Alkoholen, Toluol, Xylolen, Ester der Ameisensäure mit C₁-C₄ Alkoholen und Ester der Essigsäure mit C₁-C₄ Alkoholen verwendet. Besonders bevorzugt wird mindestens ein Antilösungsmittel ausgewählt aus der Gruppe von Wasser, Acetonitril, Methanol, Ethanol, Isopropanol, 1-Butanol, Toluol und Ethylacetat eingesetzt. In einer weiteren bevorzugten Ausführungsform der Erfindung liegt ein Antilösungsmittel vor und kein Gemisch der oben genannten Antilösungsmittel.

Bei einer weiteren bevorzugten Ausführungsform der Erfindung ist das Gewichtsmengenverhältnis zwischen dem mindestens einen amidischen Lösungsmittel und dem mindestens einen Antilösungsmittel zwischen 10:1 und 1:1, besonders bevorzugt zwischen 5:1 und 2:1 und ganz besonders bevorzugt bei etwa 4:1.

Das mindestens eine amidische Lösungsmittel kann auch bereits mit dem mindestens einen Antilösungsmittel und der Verbindung gemäß Formel (I) in der Eduktmischung verdünnt sein, soweit die eingesetzten Lösungsmittel unter den Reaktionsbedingungen inert sind und vor der Kristallisation des Solvats ein homogener Zustand durch bspw. entsprechende Temperaturerhöhung erreicht wird.

Vor Zugabe des mindestens einen Antilösungsmittels zur Eduktmischung können solche inerten Lösungsmittel, die z.B. auch mit der Lösung der Verbindung der Formel (II) in das Reaktionsgemisch gelangten, durch geeignete Maßnahmen entfernt werden, z.B. durch Destillation ggfs. unter reduziertem Druck.

Die Solvat-Kristallisation nach dem oben beschriebenen erfindungsgemäßen Verfahren wird vorzugsweise bei Temperaturen von -20 bis +30 °C und besonders bevorzugt bei Temperaturen von -10 bis +20 °C im Allgemeinen unter Normaldruck durchgeführt.

Alternativ ist es jedoch auch möglich, im Vakuum oder unter Überdruck im Autoklav zu arbeiten. Die Reaktionszeit kann, in Abhängigkeit von der Ansatzgröße, amidischen Lösungsmittel(n), Antilösungsmittel(n) und der Temperatur, in einem Bereich zwischen einer Stunde und mehreren Stunden gewählt werden. Die Filtration kann in einem dem Fachmann bekannten Filtrationsapparat durchgeführt werden wie beispielsweise einer Drucknutsche oder einer Zentrifuge. Die Trocknung kann in einem dem Fachmann bekannten Trocknungsapparat durchgeführt werden wie beispielsweise einem horizontalen oder vertikalen Mischertrockner oder einem Nutschtrockner. Die Trocknung erfolgt in einem Temperaturbereich von 20 bis 100 °C, bevorzugt in einem Temperaturbereich von 60 bis 80 °C. Die Trocknung erfolgt in einem Druckbereich von 1 bis 100 mbar, bevorzugt in einem Druckbereich von 5 bis 20 mbar.

Vorzugsweise wird die für das oben beschriebene Verfahren verwendete Eduktmischung bestehend aus einer Verbindung der oben genannten Formel (I) zusammen mit mindestens einem amidischen Lösungsmittel hergestellt durch ein Verfahren bei dem eine Verbindung der Formel (II) 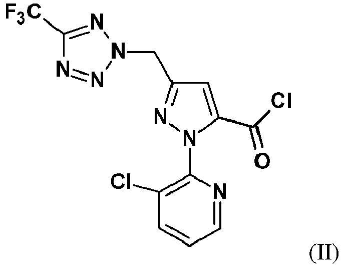 mit einer Verbindung der Formel (III) 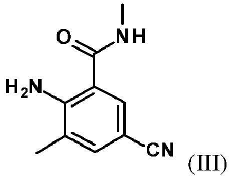 in Gegenwart eines amidischen Lösungsmittels zur Reaktion gebracht werden.

Die Kupplungsreaktion kann gegebenenfalls in Gegenwart eines Katalysators durchgeführt werden. Beispielsweise genannt seien 4-Dimethylaminopyridin, 1-Hydroxy-benzotriazol. Zusätzliche Säurebindemittel sind für diese Umsetzung nicht erforderlich.

Die Kupplungsreaktion wird weiter bevorzugt so ausgeführt, dass die Verbindung der Formel (III) in dem amidischen Lösungsmittel in äquimolaren Mengen oder in einem leichten Überschuss von 1.0 - 1.2 Moläquivalenten bezogen auf die Verbindung der Formel (II) vorgelegt wird. Die Verbindung der Formel (II) wird dann über einen Zeitraum von vorzugsweise 1 bis 10 Stunden, bevorzugt 2 bis 5 Stunden als Lösung vorzugsweise in einem inerten organischen Lösungsmittel oder als Schmelze bei einer Temperatur von vorzugsweise -10 bis +50 °C, besonders bevorzugt 0 bis 40 °C, ganz besonders bevorzugt 10 bis 30 °C zudosiert. Diese Reaktion wird im Allgemeinen unter Normaldruck durchgeführt. Alternativ ist es jedoch auch möglich, im Vakuum oder unter Überdruck im Autoklav zu arbeiten. Nach Ende der Nachreaktionszeit wird das inerte Lösungsmittel wie oben bereits beschrieben vorzugsweise entfernt.

Als amidische Lösungsmittel für diese Kupplungsreaktion werden die gleichen amidischen Lösungsmittel eingesetzt, wie für die Eduktmischung oben beschrieben.

Die Verbindung der Formel (II) kann für diese Kupplungsreaktion als Feststoff bzw. als Schmelze eingesetzt werden. Der Einsatz der Verbindung der Formel (II) gelöst in einem inerten organischen Lösungsmittel ist jedoch bevorzugt. In diesem Zusammenhang wird die Verbindung der Formel (II) in einem inerten organischen Lösungsmittel gelöst, welches vorzugsweise ausgewählt ist aus der Gruppe von aliphatischen, alicyclischen und aromatischen Kohlenwasserstoffen, wie beispielsweise Petrolether, Hexan, Heptan, Cyclohexan, Methylcyclohexan, Benzol, Toluol, Xylol und Decalin; halogenierte Kohlenwasserstoffe, wie beispielsweise Chlorbenzol, Dichlorbenzol, Dichlormethan, Chloroform, Tetrachlormethan, Dichlorethan und Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan und Anisol; Ketone, wie Aceton, Butanon, Methylisobutylketon und Cyclohexanon; Nitrile, wie Acetonitril, Propionitril, n- oder i-Butyronitril und Benzonitril; Amide, wie N,N-Dimethylformamid, N,N-Dimethylacetamid, N-Methylformanilid, N-Methylpyrrolidon und Hexamethylphosphorsäuretriamid sowie deren Gemische verwendet. Besonders bevorzugt ist die Verwendung von Toluol.

Verbindungen der Formel (III) sind bekannt oder können nach allgemeinen Synthesemethoden hergestellt werden (vgl. z. B. Baker et al. J. Org. Chem. 1952, 149-153; G. Reissenweber et al., Angew. Chem 1981, 93, 914-915, P.J. Montoya-Pelaez, J. Org. Chem. 2006, 71, 5921-5929; F. E. Sheibley, J. Org. Chem. 1938, 3, 414-423, WO 2006023783 A1).

In einer bevorzugten Ausführungsform der Erfindung wird eine Verbindung der Formel (II) dadurch hergestellt, dass eine Verbindung der Formel (IV) 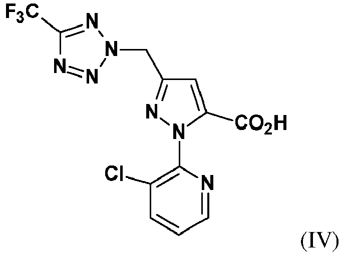 mit einem Säurehalogenidbildner ausgewählt aus der Gruppe von Phosgen, Phosphortribromid, Phosphortrichlorid, Phosphorpentachlorid, Phosphoroxychlorid und Thionylchlorid in Gegenwart eines inerten organischen Lösungsmittels zur Reaktion gebracht wird.

Der Säurehalogenidbildner wird vorzugsweise ausgewählt aus der Gruppe von Phosgen, Mesylchlorid und Thionylchlorid. Besonders bevorzugt ist Thionylchlorid.

Als inertes organisches Lösungsmittel werden die oben, für die Herstellung der Eduktmischung ausgehend von den Verbindungen der Formel (II) und (III), bereits genannten inerten organischen Lösungsmittel eingesetzt. Besonders bevorzugt ist die Verwendung von Toluol.

Dieser Verfahrenschritt wird vorzugsweise bei Temperaturen von +20 bis +100 °C und besonders bevorzugt bei Temperaturen von +50 bis +75 °C im Allgemeinen unter Normaldruck durchgeführt. Die Reaktionszeit liegt, in Abhängigkeit von der Ansatzgröße, verwendeten Edukten und Temperatur, in einem Bereich zwischen einer Stunde und fünf Stunden vorzugsweise zwischen einer Stunde und drei Stunden.

Die Pyrazolcarbonsäuren der Formel (IV) sind bekannt oder können nach bekannten Verfahren erhalten werden (vgl. z.B. WO2011/157664 A1). Aufgrund der Tetrazolgruppe liegen Pyrazolcarbonsäuren der Formel (IV) in der Regel als Mischungen aus verschiedenen Regioisomeren vor.

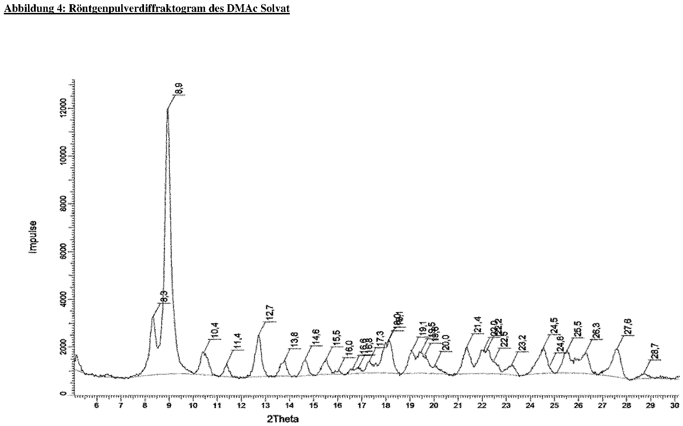

Ein weiterer Gegenstand der vorliegenden Erfindung bezieht sich auf ein kristallines N-N-Dimethylacetamid Solvat einer Verbindung der Formel (I) 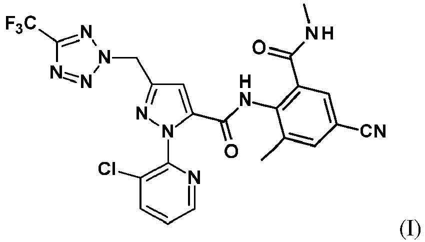 welches im Röntgenpulverdiffraktogramm bei einer Temperatur von 25 °C bei Verwendung von Cu Kα Strahlung mindestens folgende Reflexlagen aufweist: 8.3, 8.9, 14.6, besonders bevorzugt mindestens folgende Reflexlagen aufweist: 8.3, 8.9, 10.4, 14.6, 15.5, ganz besonders bevorzugt mindestens folgende Reflexlagen aufweist: 8.3, 8.9, 10.4, 12.7, 14.6, 15.5, 27.6 (angegeben als 2 Theta/° Wert ± 0.2°). Vorzugsweise weist das erfindungsgemäße kristalline N-N-Dimethylacetamid Solvat einer Verbindung der Formel (I) (abgekürzt DMAc Solvat) die in der Tabelle 3 angegebenen Reflexlagen (2 Theta) auf. Das Röntgenpulverdiffraktogramm des DMAc Solvats ist auch in der Abbildung 4 wiedergegeben.

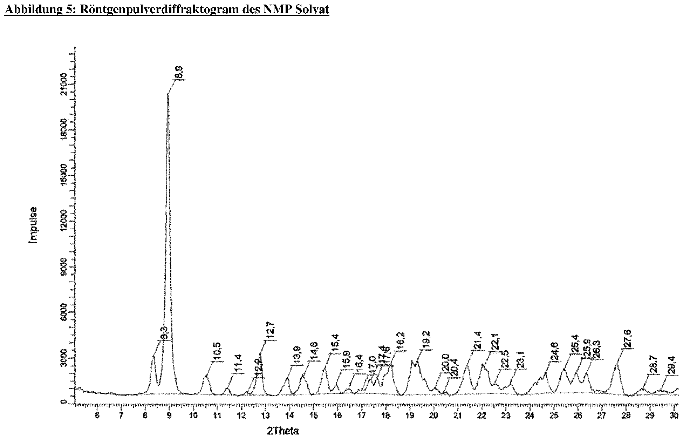

Ein weiterer Gegenstand der vorliegenden Erfindung bezieht sich auf ein kristallines N-Methyl-2-pyrrolidon-Solvat einer Verbindung der Formel (I) 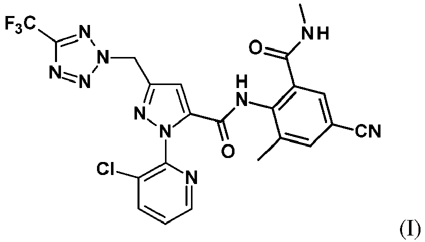 welches im Röntgenpulverdiffraktogramm bei einer Temperatur von 25 °C bei Verwendung von Cu Kα Strahlung mindestens folgende Reflexlagen aufweist: 8.3, 8.9, 14.6, besonders bevorzugt mindestens folgende Reflexlagen aufweist: 8.3, 8.9, 10.5, 14.6, 15.4, ganz besonders bevorzugt mindestens folgende Reflexlagen aufweist: 8.3, 8.9, 10.5, 12.7, 14.6, 15.4, 27.6 (angegeben als 2 Theta/° Wert ± 0.2°). Vorzugsweise weist das erfindungsgemäße kristalline N-Methyl-2-pyrrolidon Solvat einer Verbindung der Formel (I) (abgekürzt NMP Solvat) die in der Tabelle 3 angegebenen Reflexlagen (2 Theta) auf. Das Röntgenpulverdiffraktogramm des NMP Solvats ist auch in der Abbildung 5 wiedergegeben.

Alle Röntgenpulverdiffraktometrie-Daten vom DMAc und NMP Solvat wurden an einem Bruker D2 PHASER Diffraktometer mit einem LynxEye Detektor unter Verwendung von Cu Kα Strahlung der Wellenlänge 1.5418 Å bei 25 °C gemessen. Die Daten wurden in einer horizontalen Bragg-Brentano (θ/2θ) Geometrie zwischen 5 und 30° (2θ) in 0.0264119° Schritten bei 0.5 s/Schritt aufgenommen. Die Röntgenröhre wurde bei 30 kV und 10 mA betrieben. Alle Röntgenreflexionen werden als 2θ (theta) Werte (Peakmaxima) mit einer Auflösung von ±0.2° angegeben.

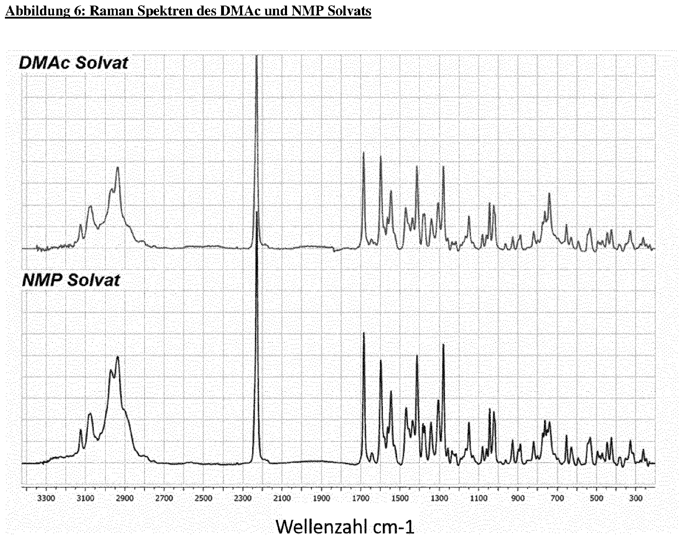

Das kristalline N-N-Dimethylacetamid Solvat und das kristalline N-Methyl-2-pyrrolidon Solvat einer Verbindung der Formel (I) kann außerdem durch IR- und Raman-Spektroskopie charakterisiert werden. Die entsprechenden Raman- und IR-Spektren sind in Abbildung 6, 7 und 8 abgebildet.

Alle Raman-Spektren der Solvate wurden mit einem Kaiser Raman RXN2 Spektrometer bei 25°C unter Verwendung einer Faser-gekoppelten Sonde zur in situ Detektion aufgenommen. Das verwendete System war ausgestattet mit einer MR Sonde zur kontaktlosen Bestimmung. Es wurde ein NIR Kaiser Invictus Laser (785 nm) mit einer Emission von 450 mW verwendet. Der Spektralbereich dieses Systems umfasst +100 bis +3425 cm⁻¹ bei einer Auflösung von 4 cm⁻¹. Die iC Raman Software der Firma Mettler Toledo wurde zur Konfiguration der Instrumente, der Datenaufzeichnung und der Datenauswertung verwendet.

Die IR Spektren der Solvate wurden durch FTIR Analytik im Spektralbereich von 400 bis 4000 cm⁻¹ mit einem Bruker Platinum ATR Tensor II und einer Auflösung von 4 cm⁻¹ aufgenommen. Die Opus Software der Firma Bruker wurde zur Konfiguration der Instrumente, der Datenaufzeichnung und der Datenauswertung verwendet.

Die IR- und Raman-Spektren der DMAc und NMP Solvate, enthalten die Banden, die der Tabelle 4 aufgelistet sind.

Vorzugsweise weist das Raman-Spektrum des DMAc Solvats mindestens folgende charakteristische Banden auf: 3126, 1685, 1340, besonders bevorzugt folgende Banden: 3126, 3026, 1685, 1340, 1306, ganz besonders bevorzugt folgende Banden: 3126, 3026, 2972, 1685, 1340, 1306, 963 (angegeben als Bande [cm⁻¹]; jeweils± 2° cm⁻¹).

Vorzugsweise weist das IR-Spektrum des DMAc Solvats mindestens folgende charakteristische Banden auf: 3234, 1525, 926, besonders bevorzugt folgende Banden: 3234, 3124, 1525, 1496, 926, ganz besonders bevorzugt folgende Banden: 3234, 3124, 3078, 1525, 1496, 1016, 926 (angegeben als Bande [cm⁻¹]; jeweils± 2° cm⁻¹).

Vorzugsweise weist das Raman-Spektrum des NMP Solvats mindestens folgende charakteristische Banden auf: 3125, 1684, 1342, besonders bevorzugt folgende Banden: 3125, 3024, 1684, 1342, 1305, ganz besonders bevorzugt folgende Banden: 3125, 3024, 2973, 1684, 1342, 1305, 963 (angegeben als Bande [cm⁻¹]; jeweils± 2° cm⁻¹).

Vorzugsweise weist das IR-Spektrum des NMP Solvats mindestens folgende charakteristische Banden auf: 3234, 1525, 926, besonders bevorzugt folgende Banden: 3234, 3124, 1525, 926, 848, ganz besonders bevorzugt folgende Banden: 3234, 3124, 3078, 1525, 1016, 926, 848 (angegeben als Bande [cm⁻¹]; jeweils± 2° cm⁻¹).

Im Folgenden wird das erfindungsgemäße Verfahren, durch welches u.a. die neuen kristallinen DMAc und NMP Solvate der Verbindung der Formel (I) erhalten werden, näher beschrieben:

### Herstellbeispiele

Die folgenden Herstellbeispiele illustrieren die Erfindung, ohne sie zu beschränken.

### Beispiel 1

### Isomerengemisch von 1-(3-chloropyridin-2-yl)-3-[(-5-(trifluoromethyl)-2H-tetrazol-2-yl)methyl]-1H-pyrazol-5-carbonylchlorid (Hauptisomer) und 1-(3-chloropyridin-2-yl)-3-{[5-(trifluoromethyl)-1H-tetrazol-1-yl]methyl}-1H-pyrazol-5-carbonylchlorid (Nebenkomponente)

50.0 g 1-(3-chloropyridin-2-yl)-3-[(5-(trifluoromethyl)-2H-tetrazol-2-yl)methyl]-1H-pyrazol-5-carbonsäure und 1-(3-chloropyridin-2-yl)-3-{[5-(trifluoromethyl)-1H-tetrazol-1-yl]methyl}-1H-pyrazol-5-carbonsäure als 95:5 Isomerengemisch wurden in 200.0 g Toluol vorgelegt und auf 75 °C erhitzt. 17.1 g Thionylchlorid wurden innerhalb von 1h zudosiert und anschließend 1h bei 75 °C nachgerührt. Nach beendeter Reaktion wurde der Überschuss Thionylchlorid zusammen mit einer Teilmenge Toluol bei 70 °C und 150 mbar abdestilliert (27.0 g Destillat). Die Lösung wurde mit frischem Toluol auf ein Gesamtgewicht von 240.0 g aufgefüllt um eine ca. 20 Gew% Lösung des Produkts als 95:5 Isomerengemisch zu erhalten, die so in der nächsten Stufe eingesetzt wurde.

### Beispiel 2

### Isomerengemisch von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid (Hauptisomer) und 1-(3-chloropyridin-2-yl)-N-[4-cyano-2-methyl-6-(methylcarbamoyl)phenyl]-3-{[5-(trifluoromethyl)-1H-tetrazol-1-yl]methyl}-1H-pyrazol-5-carboxamid (Nebenkomponente)

### a) Herstellung einer Lösung von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamide und Isomer in N,N-Dimethylacetamid (DMAc)

25.3 g 2-Amino-5-cyano-N-3-dimethylbenzamid wurden in 123.0 g DMAc gelöst und anschließend bei 65 °C und 20 mbar 20.0 g DMAc abdestilliert. Die Mischung wurde auf 10 - 15 °C gekühlt und die zuvor hergestellte 20% Lösung von 1-(3-chloropyridin-2-yl)-3-[(5-(trifluoromethyl)-2H-tetrazol-2-yl)methyl]-1H-pyrazol-5-carbonylchlorid und Isomer innerhalb von 1h zudosiert und nach beendeter Zugabe 3h bei 10 - 15 °C nachgerührt. Anschließend wurde Toluol bei 45 - 50 °C und 30 mbar abdestilliert und man erhielt eine ca. 35% w/w Lösung von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid und dem entsprechenden Isomer in DMAc.

### b) Herstellung des DMAc Solvat von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamidund Isomer

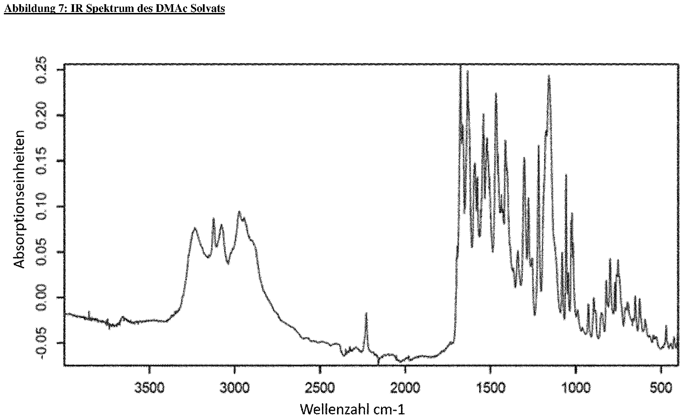

Die ca. 35% w/w Lösung von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid in DMAc wurde auf 25 - 30 °C abgekühlt und 27.0 g Methanol innerhalb von 10 Minuten zugegeben. Das Gemisch wurde über einen Zeitraum von 2 h auf 0 - 5°C abgekühlt und 1h nachgerührt. Eine Kristallprobe der Suspension zeigte rautenförmige DMAc Solvat Kristalle unter dem Lichtmikroskop. Das erhaltene DMAc Solvat zeigte die in Tabelle 3 und Abbildung 4 beschriebenen XRPD Reflexe, sowie die in Tabelle 4 und den Abbildungen 6 und 7 angegebenen Raman- und IR Spektren.

### c) Isolierung des DMAc Solvat von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid und Isomer und Trocknung

Für eine Vervollständigung der Ausbeute wurden 30.0 g Wasser innerhalb von 10 Minuten zu obiger Suspension zugegeben und 1h bei 0 - 5°C nachgerührt. Der Feststoff wurde über eine Nutsche abfiltriert, bei 0 - 5 °C mit einer Mischung aus 50.0 g DMAc und 50.0 g Wasser gewaschen und der feuchte Filterkuchen bei 80 °C und 10 mbar getrocknet. Man erhielt 66.0 g des Produkts (89% Ausbeute) als 95:5 Isomerengemisch, einer Reinheit von 95% und in kristalliner Form. Die erhaltene kristalline Form zeigte das in Tabelle 1, sowie Abbildungen 1 angegebenen charakteristische Röntgenpulverdiffraktogramm, sowie die in Tabelle 2 und den Abbildungen 2 und 3 angegebenen Raman-bzw. IR-Spektren.

### Beispiel 3

### Herstellung des DMAc Solvat von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid und Isomer unter Verwendung alternativer Anti-Solventien, optional ohne Verwendung eines Anti-Solvent

175.0 g der nach Beispiel 2a hergestellten 35% w/w Lösung von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamideund Isomer in DMAc wurden auf 30°C abgekühlt. 27.0 g eines der folgenden Lösemittel oder alternativ eines Gemisch der folgenden Lösemittel wurde innerhalb von 10 min zugegeben: Ethanol, 2-Propanol, 1-Butanol, Toluol, Xylol, Ethylacetat oder Isopropylacetat. Optional wurde kein Anti-Solvent zugegeben. Anschließend wurde innerhalb von 2h auf 0 - 5°C abgekühlt und 1h nachgerührt. Gegebenenfalls wurde die Lösung durch Zugabe einer kleinen Menge Solvat angeimpft um eine Kristallisation einzuleiten. Eine Kristallprobe der Suspension zeigte auch rautenförmige DMAc Solvat Kristalle unter dem Lichtmikroskop. Das erhaltene DMAc Solvat zeigte die in Tabelle 3 und Abbildung 4 beschriebenen XRPD Reflexe, sowie die in Tabelle 4 und den Abbildungen 6 und 7 angegebenen Raman- und IR Spektren.

### Beispiel 4

### Herstellung des NMP Solvat von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid und Isomer und Trocknung

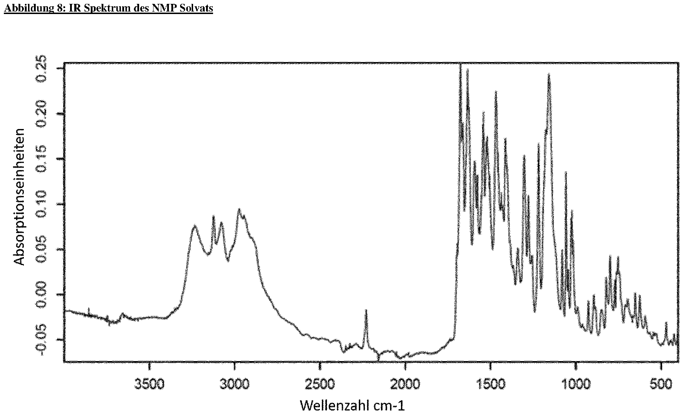

114.0 g einer nach Beispiel 2a hergestellten 35% w/w Lösung von 1-(3-chloropyridin-2-yl)-N-[4-cyano-2-methyl-6-(methylcarbamoyl)phenyl]-3-[(5-(trifluoromethyl)-2H-tetrazol-2-yl)methyl]-1H-pyrazol-5-carboxamid und Isomer in NMP wurden auf 30°C abgekühlt. 17.0 g Methanol wurden innerhalb von 10 Minuten zugegeben. Das Gemisch wurde über einen Zeitraum von 2h auf 0 - 5°C abgekühlt und 1h nachgerührt. Eine Kristallprobe der Suspension zeigte rautenförmigen NMP Solvat Kristalle unter dem Lichtmikroskop. Das erhaltene NMP Solvat zeigte die in Tabelle 3 und Abbildung 5 beschriebenen XRPD Reflexe, sowie die in Tabelle 4 und den Abbildungen 6 und 8 angegebenen Raman- und IR Spektren.

Der Feststoff wurde über eine Nutsche abfiltriert und der feuchte Filterkuchen bei 80°C und 10 mbar getrocknet. Man erhielt 31.4 g des Produkts als 95:5 Isomerengemisch in kristalliner Form. Die erhaltene kristalline Form zeigte das in Tabelle 1, sowie Abbildungen 1 angegebenen charakteristische Röntgenpulverdiffraktogramm, sowie die in Tabelle 2 und den Abbildungen 2 und 3 angegebenen Raman-bzw. IR-Spektren.

Die Solvat-Proben für die Röntgenpulverdiffraktometrie, IR- und Raman Analysen wurden nach für den Fachmann bekannten Methoden bereitgestellt.

**Tabelle 1: Röntgenpulverdiffraktometrie**

| **Reflexe [°2 Theta]** |
|---|
| **Kristalline Form** |
| 5.8 |
| 6.4 |
| 8.0 |
| 9.2 |
| 10.2 |
| 10.7 |
| 11.6 |
| 12.8 |
| 13.6 |
| 14.9 |
| 16.0 |
| 16.7 |
| 17.5 |
| 18.3 |
| 19.0 |
| 19.4 |
| 19.8 |
| 20.8 |
| 21.5 |
| 21.7 |
| 23.5 |
| 24.2 |
| 24.6 |
| 25.3 |
| 25.6 |
| 26.2 |
| 27.5 |
| 28.4 |
| 29.4 |
| 30.3 |
| 31.3 |
| 32.2 |
| 32.5 |
| 37.2 |

**Tabelle 2: IR- und Raman-Banden**

| **Kristalline Form** | **Kristalline Form** |
|---|---|
| **IR-Banden [cm⁻¹]** | **Raman-Banden [cm⁻¹]** |
| 3286 | 3073 |
| 3124 | 3059 |
| 3074 | 2998 |
| 3001 | 2952 |
| 2231 | 2927 |
| 1662 | 2232 |
| 1637 | 1663 |
| 1602 | 1639 |
| 1585 | 1603 |
| 1574 | 1574 |
| 1567 | 1543 |
| 1543 | 1528 |
| 1527 | 1512 |
| 1511 | 1466 |
| 1466 | 1442 |
| 1447 | 1435 |
| 1434 | 1424 |
| 1424 | 1386 |
| 1411 | 1334 |
| 1385 | 1274 |
| 1361 | 1246 |
| 1333 | 1229 |
| 1314 | 1219 |
| 1298 | 1190 |
| 1274 | 1159 |
| 1245 | 1139 |
| 1219 | 1116 |
| 1181 | 1081 |
| 1154 | 1056 |
| 1081 | 1043 |
| 1055 | 1029 |
| 1041 | 1022 |
| 1029 | 1009 |
| 1022 | 965 |
| 1009 | 927 |
| 991 | 903 |
| 964 | 897 |
| 955 | 882 |
| 927 | 806 |
| 902 | 772 |
| 882 | 761 |
| 830 | 746 |
| 806 | 672 |
| 796 | 638 |
| 774 | 554 |
| 758 | 539 |
| 745 | 490 |
| 729 | 475 |
| 707 | 457 |
| 693 | 435 |
| 670 | 412 |
| 635 | 402 |
| 626 | 387 |
| 592 | 367 |
| 581 | 349 |
| 573 | 339 |
| 566 | 327 |
| 553 | 295 |
| | 272 |
| | 259 |
| | 239 |
| | 219 |
| | 196 |
| | 169 |
| | 145 |
| | 119 |
| | 95 |

**Tabelle 3: Röntgenpulverdiffraktometrie**

| **Reflexe [°2 Theta]** | |
|---|---|
| **DMAc Solvat** | **NMP Solvat** |
| 8.3 | 8.3 |
| 8.9 | 8.9 |
| 10.4 | 10.5 |
| 11.4 | 11.4 |
| 12.7 | 12.2 |
| 13.8 | 12.7 |
| 14.6 | 13.9 |
| 15.5 | 14.6 |
| 16.0 | 15.4 |
| 16.6 | 15.9 |
| 16.9 | 16.4 |
| 17.3 | 17.0 |
| 18.0 | 17.4 |
| 18.1 | 17.6 |
| 19.1 | 18.2 |
| 19.5 | 19.2 |
| 19.6 | 20.0 |
| 20.0 | 20.4 |
| 21.4 | 21.4 |
| 22.0 | 22.1 |
| 22.2 | 22.5 |
| 22.5 | 23.1 |
| 23.2 | 24.6 |
| 24.5 | 25.4 |
| 24.8 | 25.9 |
| 25.5 | 26.3 |
| 26.3 | 27.6 |
| 27.6 | 28.7 |
| 28.7 | 29.4 |

**Tabelle 4: IR- und Raman-Banden**

| **DMAc Solvat** | **NMP Solvat** | **DMAc Solvat** | **NMP Solvat** |
|---|---|---|---|
| **IR-Banden [cm⁻¹]** | **IR-Banden [cm⁻¹]** | **Raman-Banden [cm⁻¹]** | **Raman-Banden [cm⁻¹]** |
| 3234 | 3234 | 3126 | 3125 |
| 3124 | 3124 | 3076 | 3077 |
| 3078 | 3078 | 3026 | 3024 |
| 2944 | 2971 | 2972 | 2973 |
| 2325 | 2228 | 2935 | 2936 |
| 1677 | 1677 | 2876 | 2887 |
| 1637 | 1637 | 2230 | 2229 |
| 1627 | 1635 | 1685 | 1684 |
| 1593 | 1593 | 1645 | 1643 |
| 1578 | 1578 | 1598 | 1598 |
| 1542 | 1542 | 1564 | 1563 |
| 1525 | 1525 | 1546 | 1546 |
| 1520 | 1520 | 1526 | 1526 |
| 1505 | 1505 | 1470 | 1469 |
| 1496 | 1468 | 1437 | 1437 |
| 1469 | 1456 | 1414 | 1413 |
| 1456 | 1436 | 1378 | 1378 |
| 1436 | 1413 | 1340 | 1342 |
| 1413 | 1340 | 1306 | 1305 |
| 1338 | 1303 | 1279 | 1279 |
| 1303 | 1278 | 1256 | 1256 |
| 1278 | 1218 | 1235 | 1235 |
| 1219 | 1158 | 1217 | 1217 |
| 1158 | 1080 | 1150 | 1150 |
| 1080 | 1057 | 1128 | 1127 |
| 1057 | 1044 | 1081 | 1080 |
| 1044 | 1027 | 1060 | 1060 |
| 1023 | 1022 | 1044 | 1044 |
| 1016 | 1016 | 1022 | 1022 |
| 926 | 926 | 963 | 963 |
| 894 | 895 | 926 | 928 |
| 851 | 848 | 893 | 893 |
| 821 | 821 | 821 | 821 |
| 799 | 799 | 799 | 801 |
| 771 | 771 | 768 | 765 |
| 752 | 752 | 740 | 739 |
| 694 | 697 | 703 | 711 |
| 651 | 651 | 653 | 694 |
| 625 | 625 | 629 | 628 |
| 589 | 594 | 593 | 593 |
| 472 | 470 | 536 | 537 |
| 424 | 424 | 493 | 494 |
| | | 471 | 472 |
| | | 446 | 446 |
| | | 424 | 424 |
| | | 384 | 382 |
| | | 352 | 353 |
| | | 329 | 328 |
| | | 263 | 262 |

**Tabelle 5: Filtrationswiderstand α**

| | **Kristalline Form** | **DMAc Solvat** |
|---|---|---|
| **Filtrationswiderstand α (in m⁻²):** | 3.0-7.0^{∗}10¹³ | 3.8^{∗}10¹¹ |

## Patentansprüche

1. Verfahren zur Herstellung einer Verbindung der Formel (I) 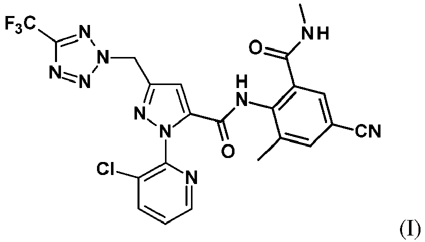 in kristalliner Form, **dadurch gekennzeichnet, dass** eine Verbindung der Formel (I) in mindestens einem amidischen Lösungsmittel gelöst wird und durch das Vorhandensein von mindestens einem Antilösungsmittel und/oder durch Temperaturerniedrigung zu einem Solvat kristallisiert und anschließend filtriert und getrocknet wird.

2. Verfahren nach Anspruch 1, **dadurch gekennzeichnet, dass** für die Kristallisation zum Solvat mindestens ein Antilösungsmittel vorhanden ist und eine Temperaturerniedrigung eingeleitet wird.

3. Verfahren nach einem der vorherigen Ansprüche, **dadurch gekennzeichnet, dass** das mindestens eine amidische Lösungsmittel ausgewählt ist aus der Gruppe von N,N-Dimethylformamid, N,N-Dimethylacetamid, N-Methylformanilid, N-Methyl-2-pyrrolidon, N-Methylcaprolactam und Hexamethylphosphorsäuretriamid.

4. Verfahren nach einem der vorherigen Ansprüche, **dadurch gekennzeichnet, dass** das mindestens eine Antilösungsmittel ausgewählt sind aus der Gruppe von Wasser, Acetonitril, C₁-C₆-Alkoholen, Toluol, Xylolen, Ester der Ameisensäure mit C₁-C₄ Alkoholen und Ester der Essigsäure mit C₁-C₄ Alkoholen.

5. Verfahren nach einem der vorherigen Ansprüche, **dadurch gekennzeichnet, dass** das Gewichtsmengenverhältnis zwischen dem mindestens einen amidischen Lösungsmittel und dem mindestens einen Antilösungsmittel zwischen 10:1 und 1:1 liegt.

6. Verfahren nach einem der vorherigen Ansprüche, **dadurch gekennzeichnet, dass** die Solvat Kristallisation bei Temperaturen von -20 bis +30 °C durchgeführt wird.

7. Verfahren nach einem der vorherigen Ansprüche, **dadurch gekennzeichnet, dass** die Verbindung der Formel (I) in einem amidischen Lösungsmittel dadurch hergestellt wird, dass eine Verbindung der Formel (II) 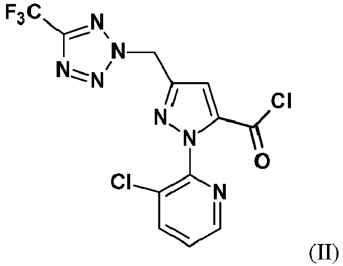 mit einer Verbindung der Formel (III) 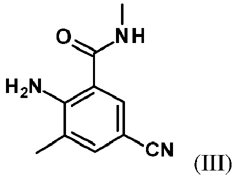 in Gegenwart eines amidischen Lösungsmittels zur Reaktion gebracht wird.

8. Verfahren nach Anspruch 7, **dadurch gekennzeichnet, dass** die Verbindung der Formel (II) dadurch hergestellt wird, dass eine Verbindung der Formel (IV) 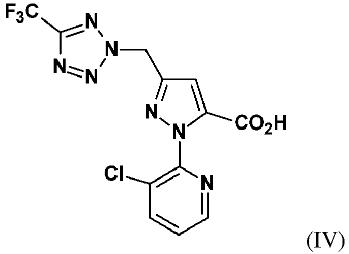 mit einem Säurehalogenidbildner ausgewählt aus der Gruppe von Phosgen, Phosphortribromid, Phosphortrichlorid, Phosphorpentachlorid, Phosphoroxychlorid und Thionylchlorid in Gegenwart eines inerten organischen Lösungsmittels zur Reaktion gebracht wird.

9. Ein kristallines N-N-Dimethylacetamid Solvat einer Verbindung der Formel (I) 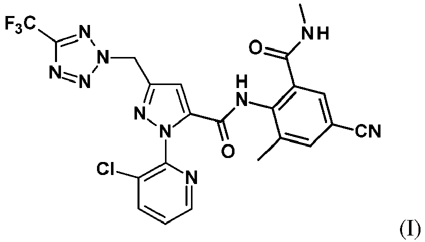 welches im Röntgenpulverdiffraktogramm bei einer Temperatur von 25°C bei Verwendung von Cu Kα Strahlung mindestens folgende Reflexlagen aufweist: 8.3, 8.9, 14.6 (angegeben als ° 2 Theta Wert ± 0.2°).

10. Ein kristallines N-N-Dimethylacetamid Solvat einer Verbindung der Formel (I) nach Anspruch 9, **dadurch gekennzeichnet, dass** ihr Raman-Spektrum mindestens folgende Banden aufweist: 3126, 1685, 1340 (angegeben als Bande [cm⁻¹]; jeweils± 2° cm⁻¹).

11. Ein kristallines N-Methyl-2-pyrrolidon Solvat einer Verbindung der Formel (I) 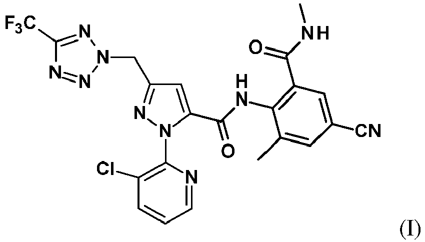 welches im Röntgenpulverdiffraktogramm bei einer Temperatur von 25°C bei Verwendung von Cu Kα Strahlung mindestens folgende Reflexlagen aufweist: 8.3, 8.9, 14.6 (angegeben als ° 2 Theta Wert ± 0.2°)

12. Ein kristallines N-Methyl-2-pyrrolidon Solvat einer Verbindung der Formel (I) nach Anspruch 11, **dadurch gekennzeichnet, dass** ihr Raman-Spektrum mindestens folgende Banden aufweist: 3125, 1684, 1342 (angegeben als Bande [cm⁻¹]; jeweils± 2° cm⁻¹).

13. Verwendung eines kristallinen N-N-Dimethylacetamid Solvats einer Verbindung der Formel (I) nach Anspruch 9 oder 10 und/oder eines kristallinen N-Methyl-2-pyrrolidon Solvats einer Verbindung der Formel (I) nach Anspruch 11 oder 12 zur Herstellung einer Verbindung der Formel (I) in kristalliner Form.

14. Verfahren nach einem der Ansprüche 1 bis 8 oder Verwendung gemäß Anspruch 13, wobei die Verbindung der Formel (I) in einer kristallinen Form erhalten wird, die **dadurch gekennzeichnet ist, dass** ihr Röntgenpulverdiffraktogramm bei einer Temperatur von 25°C und bei Verwendung von Cu Kα Strahlung mindestens folgende Reflexlagen (2 Theta) aufweist: 5.8°, 6.4°, 11.6°, 17.5°, 19.8°, 20.8°, 23.5° und 24.2° (jeweils ± 0,2°).

15. Verfahren nach einem der Ansprüche 1 bis 8 oder Verwendung gemäß Anspruch 13, wobei die Verbindung der Formel (I) in einer kristallinen Form erhalten wird, die **dadurch gekennzeichnet ist, dass** das ihr Raman-Spektrum mindestens folgende Banden [cm⁻¹] aufweist: 2928, 1663, 1386, 1334, 1022, 638 (jeweils± 2° cm⁻¹).

16. Verfahren nach einem der Ansprüche 1 bis 8 oder Verwendung gemäß Anspruch 13, wobei die Verbindung der Formel (I) in einer kristallinen Form erhalten wird, die **dadurch gekennzeichnet ist, dass** ihr IR-Spektrum mindestens folgende Banden [cm⁻¹] aufweist 3286, 1662, 1219, 1181, 1154, 1055 (jeweils± 2° cm⁻¹).
